# EUROPEAN PATENT SPECIFICATION

(11) **EP 3 478 359 B1**
(45) Date of publication and mention of the grant of the patent: **02.09.2026**
(21) Application number: 17821113.2
(22) Date of filing: 28.06.2017
(51) Int. Cl.: A61N 1/36, A61N 1/05, A61N 1/372, H04R 25/00

(54) **BIO-INSPIRED FAST FITTING OF COCHLEAR IMPLANTS**
BIOINSPIRIERTE SCHNELLE PASSUNG VON COCHLEAIMPLANTATEN
AJUSTEMENT RAPIDE BIO-INSPIRÉ D'IMPLANTS COCHLÉAIRES

(30) Priority: 30.06.2016 US 201662356588 P
(43) Date of publication of application: 08.05.2019
(73) Proprietor: MED-EL Elektromedizinische Geräte GmbH, 6020 Innsbruck (AT)
(72) Inventor: LI, Guoping, Southampton SO17 1PR (GB); MEISTER, Dirk, 6020 Innsbruck (AT); KALS, Mathias, 6094 Grinzens (AT)
(74) Representative: Lucke, Andreas
(86) International application number: PCT/US2017/039627
(87) International publication number: WO 2018/005583

(56) References cited:
- US-A1- 2007 135 862
- US-A1- 2010 152 813
- US-A1- 2011 082 519
- US-A1- 2013 079 845
- US-A1- 2013 274 827
- US-A1- 2015 045 844

## Description

### TECHNICAL FIELD

The present invention relates to hearing implant systems, and more specifically, to custom fitting of hearing implant systems such as cochlear implants.

### BACKGROUND ART

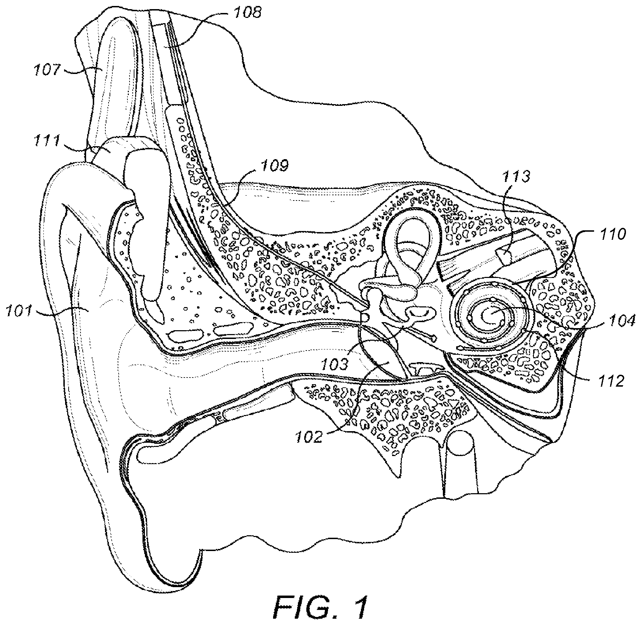

A normal ear transmits sounds as shown in Figure 1 through the outer ear **101** to the tympanic membrane (eardrum) **102,** which vibrates the ossicles of the middle ear **103** (malleus, incus, and stapes). The stapes footplate is positioned in the oval window **106** that forms an interface to the fluid filled inner ear (the cochlea) **104.** Movement of the stapes generates a pressure wave in the cochlea **104** that stimulates the sensory cells of the auditory system (hair cells). The cochlea **104** is a long narrow duct wound spirally around its central axis (called the modiolus) for approximately two and a half turns. The cochlea **104** includes an upper channel known as the scala vestibuli, a middle channel known as the scala media and a lower channel known as the scala tympani. The hair cells connect to the spiral ganglion cells of the cochlear nerve **105** that reside in the modiolus. In response to received sounds transmitted by the middle ear **103,** the fluid-filled cochlea **104** functions as a transducer to generate electric pulses which are transmitted to the cochlear nerve **105,** and ultimately to the brain.

Hearing is impaired when there are problems in the ability to transduce external sounds into meaningful action potentials along the neural substrate of the cochlea **104.** To improve impaired hearing, auditory prostheses have been developed. For example, when the impairment is related to operation of the middle ear **103,** a conventional hearing aid or middle ear implant may be used to provide acoustic-mechanical stimulation to the auditory system in the form of amplified sound. Or when the impairment is associated with the cochlea **104,** a cochlear implant with an implanted stimulation electrode can electrically stimulate auditory nerve tissue with small currents delivered by multiple electrode contacts distributed along the electrode.

Figure 1 also shows some components of a typical cochlear implant system, including an external microphone that provides an audio signal input to an external signal processor **111** where various signal processing schemes can be implemented. The processed signal is then converted into a digital data format, such as a sequence of data frames, for transmission into the implant **108.** Besides receiving the processed audio information, the implant **108** also performs additional signal processing such as error correction, pulse formation, etc., and produces a stimulation pattern (based on the extracted audio information) that is sent through an electrode lead **109** to an implanted electrode array **110.** The electrode array **110** includes multiple electrode contacts **112** (also referred to as electrode channels) on its surface that provide selective stimulation of the cochlea **104.**

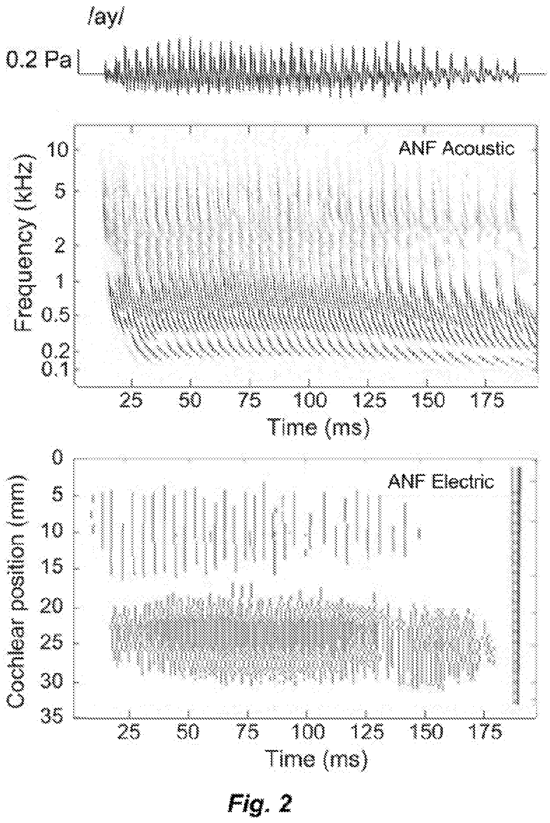

A relatively small number of electrode channels are each associated with relatively broad frequency bands, with each electrode contact **112** addressing a group of neurons with an electric stimulation pulse having a charge that is derived from the instantaneous amplitude of the signal envelope within that frequency band. Current cochlear implant coding strategies map the different sound frequency channels onto different locations within the cochlea. Figure 2 shows one example of the processing of a signal using the cochlear implant stimulation (CIS) stimulation strategy. The top of Fig. 2 shows the sound pressure characteristics of a spoken "A" (/ay/) at a sound level of 67.2 dB. The middle waveform in Fig. 2 shows a normal healthy auditory system response. The bottom waveform in Fig. 2 shows a neural response of the auditory nerve fibers under CIS stimulation.

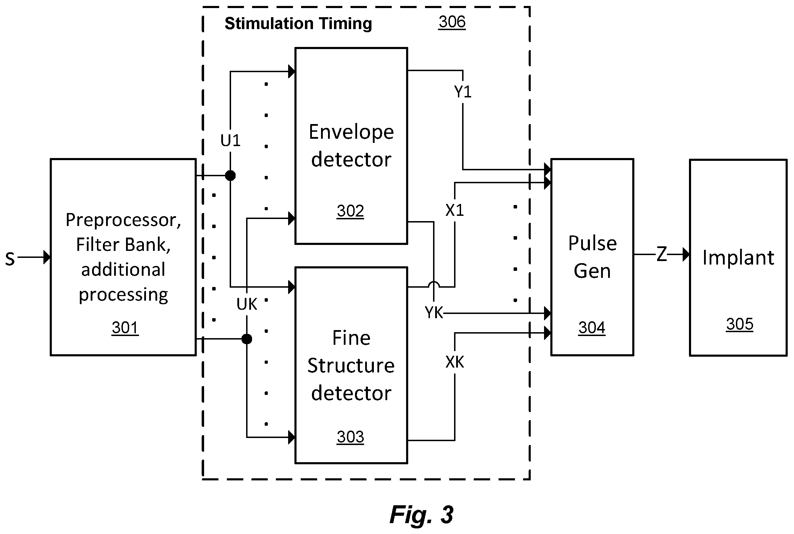

Figure 3 shows various functional blocks in a signal processing arrangement for producing electrode stimulation signals to electrode contacts in an implanted cochlear implant array according to a typical hearing implant system. A pseudo code example of such an arrangement can be set forth as:
**Input Signal Preprocessing:**
   BandPassFilter (input_sound, band_pass_signals)
**Envelope Extraction:**
   BandPassEnvelope (band_pass_signals, band_pass_envelopes)
**Stimulation Timing Generation:**
   TimingGenerate (band_pass_signals, stim_timing)
**Pulse Generation:**
   PulseGenerate (band_pass_envelopes, stim_timing, out_pulses)
The details of such an arrangement are set forth in the following discussion.

In the signal processing arrangement shown in Figure 3, the initial input sound signal is produced by one or more sensing microphones, which may be omnidirectional and/or directional. Preprocessor Filter Bank **301** pre-processes this input sound signal with a bank of multiple parallel band pass filters (e.g. Infinite Impulse Response (IIR) or Finite Impulse Response (FIR)), each of which is associated with a specific band of audio frequencies, for example, using a filter bank with 12 digital Butterworth band pass filters of 6th order, Infinite Impulse Response (IIR) type, so that the acoustic audio signal is filtered into some *K* band pass signals, *U*₁ to *U*_{K} where each signal corresponds to the band of frequencies for one of the band pass filters. Each output of sufficiently narrow CIS band pass filters for a voiced speech input signal may roughly be regarded as a sinusoid at the center frequency of the band pass filter which is modulated by the envelope signal. This is also due to the quality factor (Q ≈ 3) of the filters. In case of a voiced speech segment, this envelope is approximately periodic, and the repetition rate is equal to the pitch frequency. Alternatively and without limitation, the Preprocessor Filter Bank **301** may be implemented based on use of a fast Fourier transform (FFT) or a short-time Fourier transform (STFT). Based on the tonotopic organization of the cochlea, each electrode contact in the scala tympani typically is associated with a specific band pass filter of the Preprocessor Filter Bank **301.** The Preprocessor Filter Bank **301** also may perform other initial signal processing functions such as and without limitation automatic gain control (AGC) and/or noise reduction and/or wind noise reduction and/or beamforming and other well-known signal enhancement functions. An example of pseudocode for an infinite impulse response (IIR) filter bank based on a direct form II transposed structure is given by Fontaine et al., Brian Hears: Online Auditory Processing Using Vectorization Over Channels, Frontiers in Neuroinformatics, 3011.

The band pass signals *U*₁ to *U*_{K} (which can also be thought of as electrode channels) are output to a Stimulation Timer **306** that includes an Envelope Detector **302** and Fine Structure Detector **303.** The Envelope Detector **302** extracts characteristic envelope signals outputs Y₁, ... , Y_{K} that represent the channel-specific band pass envelopes. The envelope extraction can be represented by *Yₖ* = *LP*(|*Uₖ*|)*,* where |_{•}| denotes the absolute value and LP(.) is a low-pass filter; for example, using 12 rectifiers and 12 digital Butterworth low pass filters of 2nd order, IIR-type. Alternatively, the Envelope Detector **302** may extract the Hilbert envelope, if the band pass signals U₁, ... , U_{K} are generated by orthogonal filters.

The Fine Structure Detector **303** functions to obtain smooth and robust estimates of the instantaneous frequencies in the signal channels, processing selected temporal fine structure features of the band pass signals U₁, ... , U_{K} to generate stimulation timing signals X₁, ... , X_{K}. The band pass signals *U₁*, *..., Uₖ* can be assumed to be real valued signals, so in the specific case of an analytic orthogonal filter bank, the Fine Structure Detector **303** considers only the real valued part of *Uₖ.* The Fine Structure Detector **303** is formed of K independent, equally-structured parallel sub-modules.

The extracted band-pass signal envelopes Y₁, ..., Y_{K} from the Envelope Detector 302, and the stimulation timing signals X₁, ... , X_{K} from the Fine Structure Detector **303** are output from the Stimulation Timer **306** to a Pulse Generator **304** that produces the electrode stimulation signals Z for the electrode contacts in the implanted electrode array **305.** The Pulse Generator **304** applies a patient-specific mapping function-for example, using instantaneous nonlinear compression of the envelope signal (map law)-That is adapted to the needs of the individual cochlear implant user during fitting of the implant in order to achieve natural loudness growth. The Pulse Generator **304** may apply logarithmic function with a form-factor C as a loudness mapping function, which typically is identical across all the band pass analysis channels. In different systems, different specific loudness mapping functions other than a logarithmic function may be used, with just one identical function is applied to all channels or one individual function for each channel to produce the electrode stimulation signals. The electrode stimulation signals typically are a set of symmetrical biphasic current pulses.

It is well-known in the field that electric stimulation at different locations within the cochlea produce different frequency percepts. The underlying mechanism in normal acoustic hearing is referred to as the tonotopic principle. In cochlear implant users, the tonotopic organization of the cochlea has been extensively investigated; for example, see Vermeire et al., Neural tonotopy in cochlear implants: An evaluation in unilateral cochlear implant patients with unilateral deafness and tinnitus, Hear Res, 245(1-2), 3008 Sep 12 p. 98-106; and Schatzer et al., Electric-acoustic pitch comparisons in single-sided-deaf cochlear implant users: Frequency-place functions and rate pitch, Hear Res, 309, 3014 Mar, p. 26-35.

In some stimulation signal coding strategies, stimulation pulses are applied at a constant rate across all electrode channels, whereas in other coding strategies, stimulation pulses are applied at a channel-specific rate. Various specific signal processing schemes can be implemented to produce the electrical stimulation signals. Signal processing approaches that are well-known in the field of cochlear implants include continuous interleaved sampling (CIS), channel specific sampling sequences (CSSS) (as described in U.S. Patent No. 6,348,070), spectral peak (SPEAK), and compressed analog (CA) processing.

In the CIS strategy, the signal processor only uses the band pass signal envelopes for further processing, i.e., they contain the entire stimulation information. For each electrode channel, the signal envelope is represented as a sequence of biphasic pulses at a constant repetition rate. A characteristic feature of CIS is that the stimulation rate is equal for all electrode channels and there is no relation to the center frequencies of the individual channels. It is intended that the pulse repetition rate is not a temporal cue for the patient (i.e., it should be sufficiently high so that the patient does not perceive tones with a frequency equal to the pulse repetition rate). The pulse repetition rate is usually chosen at greater than twice the bandwidth of the envelope signals (based on the Nyquist theorem).

In a CIS system, the stimulation pulses are applied in a strictly non-overlapping sequence. Thus, as a typical CIS-feature, only one electrode channel is active at a time and the overall stimulation rate is comparatively high. For example, assuming an overall stimulation rate of 18 kpps and a 12 channel filter bank, the stimulation rate per channel is 1.5 kpps. Such a stimulation rate per channel usually is sufficient for adequate temporal representation of the envelope signal. The maximum overall stimulation rate is limited by the minimum phase duration per pulse. The phase duration cannot be arbitrarily short because, the shorter the pulses, the higher the current amplitudes have to be to elicit action potentials in neurons, and current amplitudes are limited for various practical reasons. For an overall stimulation rate of 18 kpps, the phase duration is 27 µs, which is near the lower limit.

The Fine Structure Processing (FSP) strategy by Med-El uses CIS in higher frequency channels, and uses fine structure information present in the band pass signals in the lower frequency, more apical electrode channels. In the FSP electrode channels, the zero crossings of the band pass filtered time signals are tracked, and at each negative to positive zero crossing, a Channel Specific Sampling Sequence (CSSS) is started. Typically CSSS sequences are applied on up to 3 of the most apical electrode channels, covering the frequency range up to 200 or 330 Hz. The FSP arrangement is described further in Hochmair I, Nopp P, Jolly C, Schmidt M, Schößer H, Garnham C, Anderson I, MED-EL Cochlear Implants: State of the Art and a Glimpse into the Future, Trends in Amplification, vol. 10, 201-219, 2006. The FS4 coding strategy differs from FSP in that up to 4 apical channels can have their fine structure information used. In FS4-p, stimulation pulse sequences can be delivered in parallel on any 2 of the 4 FSP electrode channels. With the FSP and FS4 coding strategies, the fine structure information is the instantaneous frequency information of a given electrode channel, which may provide users with an improved hearing sensation, better speech understanding and enhanced perceptual audio quality. See, e.g., U.S. Patent 7,561,709; Lorens et al. "Fine structure processing improves speech perception as well as objective and subjective benefits in pediatric MED-EL COMBI 40+ users." International journal of pediatric otorhinolaryngology 74.12 (2010): 1372-1378; and Vermeire et al., "Better speech recognition in noise with the fine structure processing coding strategy." ORL 72.6 (2010): 305-311.

Many cochlear implant coding strategies use what is referred to as an n-of-m approach where only some number n electrode channels with the greatest amplitude are stimulated in a given sampling time frame. If, for a given time frame, the amplitude of a specific electrode channel remains higher than the amplitudes of other channels, then that channel will be selected for the whole time frame. Subsequently, the number of electrode channels that are available for coding information is reduced by one, which results in a clustering of stimulation pulses. Thus, fewer electrode channels are available for coding important temporal and spectral properties of the sound signal such as speech onset.

Contemporary coding strategies were developed to code the spectral structure of sounds which provides sufficient cues for speech understanding. However, the complex time-place patterns observed in the intact ear cannot yet be replicated. This is also due to technical limitations as for example the channel crosstalk between electrode channels which imposes strong limitations on electrically evoked neuronal excitation patterns.

The evaluation of sound quality and speech intelligibility for the purposes of a hearing prosthesis is a complex task that is connected to many perceptual factors. The processing of the auditory system from the outer ear to the auditory nerve fibers can be represented in one or more neural models such as the neurograms shown in Fig. 2 where the x-axis represents time and the y-axis logarithmically represents center frequency of the auditory nerve fiber. Neural models can be used to efficiently predict the intelligibility aspects that relate to the first parts of the auditory pathway.

The literature in the field has proposed various speech evaluation tools. Back in 1947, French and Steinberg (Factors Governing the Intelligibility of Speech Sounds, Journal of the Acoustical Society of America, vol. 19, no. 1, pp. 90-119) proposed an articulation index (AI) to evaluate speech intelligibility of an audio signal purely as a function of the signal-to-noise-ratio (SNR) dependent on a specific threshold of hearing in twenty frequency bands. In each band the chosen SNR is used to model the overall sound quality, which can be adapted to specific hearing losses.

Bondy et al., Predicting Speech Intelligibility from a Population of Neurons, Advances in Neural Information Processing Systems, vol. 16, 2003 described a Neural Articulation Index (NAI) as a variation of the AI based on a weighted sum of the SNR of the firing rates in seven frequency bands of a neurogram..

Elhilali et al., A Spectro-Temporal Modulation Index (STMI) for Assessment of Speech Intelligibility, Speech Communication, vol. 41, no. 2, pp. 331-348, 2003 described using a Spectro-Temporal Modulation Index to evaluate the quality of an auditory model to spectro-temporal modulations under different distortions such as noise, reverberations etc. and attempted to predict speech intelligibility under the influence of these distortions using simple averaging.

Hines and Harte, Speech Intelligibility from Image Processing, Speech Communication, vol. 52, no. 9, pp. 736-752, 2010
proposed using an image processing technique known as Structural Similarity Index Measure (SSIM, or later NSIM - neurogram similarity index measure) developed by Wang et al. Image Quality Assessment: From Error Visibility to Structural Similarity, IEEE Transactions on Image Processing, vol. 13, no. 4, pp. 600-612, 2004 which regarded neurograms as images and assessed the similarity between them.

Current comparison methods for neurograms (or related neural response models) such as NI, NIT, STMI, SSIM and NSIM focus on predicting speech intelligibility in the presence of noise and other signal distortions. They try to estimate the overall quality in the neural representation of a given sound. The quality indexes NI, NIT, STMI are based on average properties of neurograms which are too coarse to be effective in capturing perceptual aspects. Also they do not allow for an adequate comparison between different neurograms which is important when designing stimulation strategies. The NSIM by Hines regards neurograms as images and attempts to predict intelligibility by comparing a degraded neurogram with a reference neurogram under normal hearing conditions. All these approaches do not exploit all relevant information coded in the temporal sequence of auditory neuronal spike trains and are inspired by engineering applications which do not necessarily fit the complex framework of human sound perception.

For an audio prosthesis such as a cochlear implant to work correctly, some patient-specific operating parameters need to be determined in a fit adjustment procedure where the type and number of operating parameters are device dependent and stimulation strategy dependent. Possible patient-specific operating parameters for a cochlear implant include:
- THR₁ (lower detection threshold of stimulation amplitude) for Electrode 1
- MCL₁ (most comfortable loudness) for Electrode 1
- Phase Duration for Electrode 1
- THR₂ for Electrode 2
- MCL₂ for Electrode 2
- Phase Duration for Electrode 2
- Pulse Rate
- Number of fine structure channels
- Compression
- Parameters of frequency->electrode mapping
- Parameters describing the electrical field distribution
These patient-specific operating parameters are saved in a file referred to as a fit map. A given system may have multiple patient-specific fit maps for different listening environments; for example, there may be one fit map for a quiet environment and a different fit map for a noisy environment. The better the fit map, the more closely the hearing experience from the electrical stimulation signals resembles the natural acoustic hearing experience of unimpaired individuals.

One common method for fit adjustment is to behaviorally find the threshold (THR) and most comfortable loudness (MCL) value for each separate electrode contact. See for example, Rätz, Fitting Guide for First Fitting with MAESTRO 2.0, MED-EL, Fürstenweg 77a, 6020 Innsbruck, 1.0 Edition, 2007. AW 5420 Rev. 1.0 (English_EU). Other alternatives/extensions are sometimes used with a reduced set of operating parameters; e.g. as suggested by Smoorenburg, Cochlear Implant Ear Marks, University Medical Centre Utrecht, 2006; and U.S. Patent Application 20060235332. Typically each stimulation channel is fitted separately without using the information from already fitted channels. The stimulation current on a given electrode typically is increased in steps from zero until the MCL or THR is reached.

One approach for an objective measurement of MCLs and THRs is based on the measurement of the ECAPs (Electrically Evoked Compound Action Potentials), as described by Gantz et al., Intraoperative Measures of Electrically Evoked Auditory Nerve Compound Action Potentials, American Journal of Otology 15 (2):137-144 (1994). In this approach, a recording electrode in the scala tympani of the inner ear is used. The overall response of the auditory nerve to an electrical stimulus is measured very close to the position of the nerve excitation. This neural response is caused by the super-position of single neural responses at the outside of the axon membranes. The amplitude of the ECAP at the measurement position is typically in the ranges of µV. When performing objective measurements such as ECAP measurements in existing cochlear implant systems, usually each electrode contact of the implantable electrode array is scanned separately, increasing the stimulation signal current on an electrode contact in steps from zero or a very low level until an ECAP response is detected. Other objective measurement approaches are also known, such as electrically evoked stapedius reflex thresholds (eSRT).

Once the fit parameters such as MCL and THR are initially established based on objective measurements, then an audiologist can further fine tune the fit map based on their experience and any available subjective feedback from the individual patient to modify the existing fit map by scaling, tilting, smoothing, or changing the shape of the fit map. However, the fitting audiologist needs to have many years of clinical experience and the fitting process can be quite time consuming. It is not trivial to test even some of the many possible adjustment combinations. In addition, patient feedback is not always available; for example, when the patient is a small child.

United States Patent Publication 20140294188 describes using a similarity index between a normal hearing neural response model and an impaired neural response model, but there is no teaching of applying that approach to automatic or fast fitting for cochlear implant systems.
US 2011/0082519 A1 discloses a system and method of fitting a cochlear implant of a patient includes analyzing data of one or more previously fitted cochlear implant users. Predicted fitting data for the patient is provided based on the analysis. Stimulation parameters of the cochlear implant are adjusted based, at least in part, on the predicted fitting data. Further steps are suggested to minimize the prediction error.

US 2013/0274827 A1 discloses a method of adjusting an established initial operational settings profile, the profile having two or more operational setting values for a speech processor of a recipient's cochlear implant, comprising: setting one or more profile adjustment functions with one or more function parameters; and modifying concurrently said two or more operational setting values in said operational settings profile using each of said set profile adjustment functions.

US 2007/135862 A1 discloses a method for fitting a multimodal hearing system for a recipient comprising: determining a desired perception for an input signal; receiving a measurement of a perception evoked by applying to the recipient one or more stimulation signals that correspond to the input signal, wherein the one or more stimulation signals applied using two or more stimulation modes, and each stimulation signal is determined using stimulus mode weighting; and adjusting one or more of the stimulus mode weightings based on the difference between the measured evoked perception and the desired perception. The method may be implemented in a clinical program system or a computer program product on a computer readable medium.

### SUMMARY

The present invention provides a method according to claim 1 and a computer-readable medium according to claim 7. Preferred embodiments are defined in the dependent claims.

### BRIEF DESCRIPTION OF THE DRAWINGS

Figure 1 shows anatomical structures of a typical human ear with a cochlear implant system.
Figure 2 shows an example of signal processing using the cochlear implant stimulation (CIS) stimulation strategy
Figure 3 shows various functional blocks in a signal processing arrangement for a typical cochlear implant system
Figure 4 shows a block diagram of a cochlear implant fitting system according to one specific embodiment of the present invention.
Figure 5 shows various steps in a process for adjusting hearing implant operating parameters according to an embodiment of the present invention.

### DETAILED DESCRIPTION

Embodiments of the present invention are directed to automatic and/or fast fitting that combines objective measurements such as ECAP and ESRT with neural response models for normal hearing and for electric stimulation.

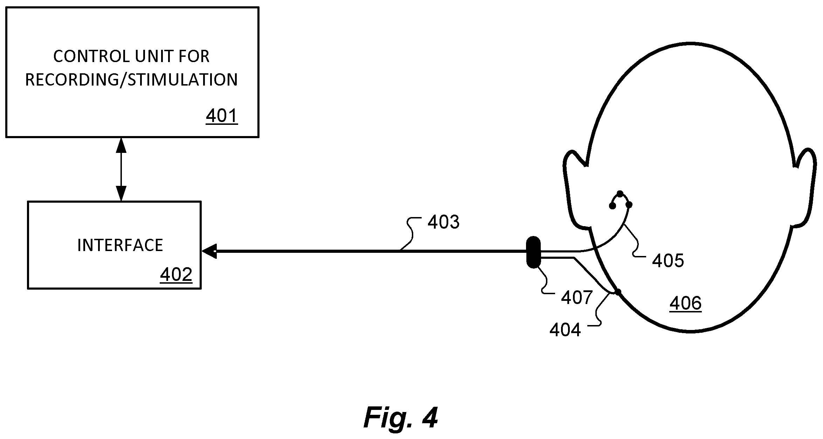

Figure 4 shows a block diagram of a cochlear implant fitting system according to an embodiment of the present invention. Control Unit **401** for Recording and Stimulation, for example, a Med-El Maestro Cochlear Implant (CI) system, generates stimulation signals and analyzes response measurements. Connected to the Control Unit **401** is an Interface Box **402,** for example, a Diagnostic Interface System such as the DIB II conventionally used with the Maestro CI system that formats and distributes the input and output signals between the Control Unit **401** and the system components implanted in the Patient **406.** For example, as shown in Fig. **4****,** there may be an Interface Lead **403** connected at one end to the Interface Box **402** and at the other end having Electrode Plug **407** that then divides into a Cochlear Implant Electrode **404** and an Extra-Cochlear Ground Electrode **405.** After delivering a stimulation pulse, a Cochlear Implant Electrode **404** may be used as a sensing element to determine current and voltage characteristics of the adjacent tissue.

The Control Unit **401** is configured to perform objective response measurements, e.g., such as ECAP/ESRT sensed by the Cochlear Implant Electrode **404,** following delivery of preliminary electrical stimulation signals to the electrode contacts in the Cochlear Implant Electrode **404** so as to determine a preliminary fit map that characterizes preliminary patient-specific operating parameters for the hearing implant system. Then, the Control Unit **401** or some other separate module (not shown) produces at least one adjusted fit map that characterizes adjusted patient-specific operating parameters for the hearing implant system based on using the preliminary fit map to constrain an implant neural response model to best fit a normal hearing neural response model.

The neural response models reflect the understanding that cochlear implants are intended to produce neural response patterns to the electrical stimulation signals which are similar to the neural responses from normal-hearing with acoustic stimuli. And it as discussed above, it is known that the neural response patterns produced by cochlear implants depend on the parameters of the electric stimuli defined in a map such as the MCL/THR levels and stimulation rate, as well as the properties of the surviving cochlear neurons such as the size of surviving population, distribution and health status. It is these parameters that are captured by the neural response models. Fitting can then be regarded as a process of minimizing the difference between the respective neural models. With similar loudness, the map that produces the greatest similarity between neural response patterns with acoustic stimuli and patterns with electric stimuli should be tried first.

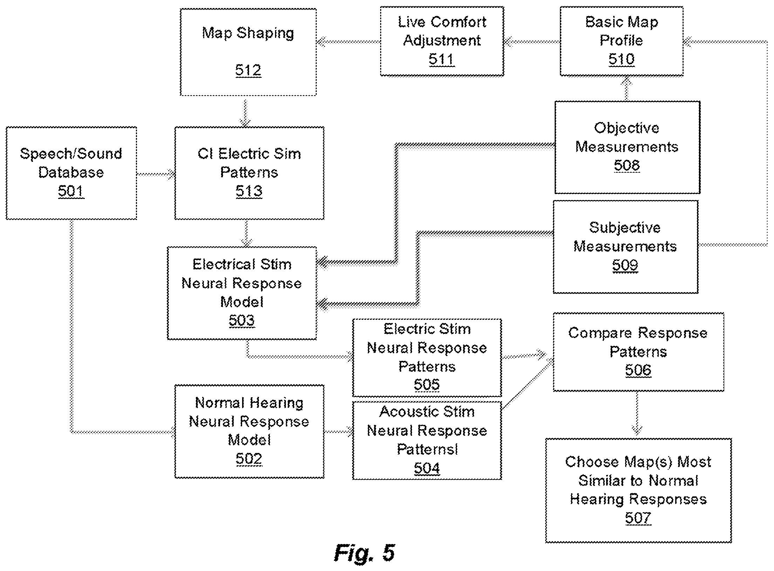

Figure 5 shows various logical steps in a process for adjusting hearing implant operating parameters according to an embodiment of the present invention using a fitting system such as the one shown in Figure 4. A speech/sound database **501** stores data for a normal hearing neural response model **502** and cochlear implant electrical stimulation patterns **513** for an electric stimulation neural response model **503,** which respectively define an acoustic stimulation neural response pattern **504** and electric stimulation neural response patterns **505.**

The electric stimulation neural response model **503** and the electric stimulation neural response patterns **505** are constrained by objective measurements **508** such as ECAP/ESRT, and any available subjective measurements **509.** For example, an ECAP loudness growth function may indicate the health status of the neurons at a particular channel for a patient. The objective measurements **508** and subjective measurements **509** also form the basis for an initial basic map profile **510** of estimated MCL/THR levels, where any non-measured channels can be interpolated. From the basic map profile **510,** the global levels of the MCL/THR can be adjusted in a live comfort adjustment **511** until the patients are comfortable to loud sounds. For infants, this can be determined by observation of the patient so reactions such as eye-blinking. Then map shaping **512** varies (e.g., randomly) the different map parameters in the CI electric stimulation patterns **513** such as MCL/THR, stimulation rate, number of active channels, pulse shape and stimulation mode to provide a number of n different maps with the constraint that the overall loudness between different maps remains similar. The map shaping change of the map parameters can also be controlled by a generic algorithm, for example, applying a set of geometric changing blocks, such as scaling, tilting and curvature (making the overall profile shape more or less curvy) within a certain percentage range e.g. by ± 15%. In some embodiments, the patient's perception performance characteristics such as aided threshold, speech or phoneme recognition rate may also be used as a further constraint.

The electric stimulation neural response patterns **505** from each of the n different maps are compared to the acoustic stimulation neural response pattern **504** using data from the speech/sound database **501** for a given sound environment such as in noise or music. The comparing can be based on using a similarity index calculation of the two response patterns such as described in Drews M. et al., The Neurogram Matching Similarity Index (NMSI) for Assessment of Similarities among Neurograms IEEE International Conference on Acoustics, Speech and Signal Processing (ICASSP) 2013, pp. 1162-1166; and in Drews M. et al., A Neurogram Matching Similarity Index for Assessment of Audio Quality, In Sound Quality Conference Vienna, 2013; which are.

The map for which the electric stimulation neural response pattern **505** is closest to the normal hearing acoustic stimulation neural response pattern **504** is chosen **507**. For different hearing environments, different optimised maps can be created and automatically activated by the signal processor or manually activated by the patient using a remote control. The fitting audiologist and/or the patient may also get an indication in a fitting dialogue about the direction of map change that provides a higher similarity index for the models used. For example, tilting a map -5% towards lower frequency may give a higher similarity index. And the audiologist/patient can then optionally further adjust the map to produce a higher similarity index.

Improved fitting arrangements such as those described above provide a rapid automatic or semi-automatic fitting and/or fine-tuning of the cochlear implant to identify the best settings for the patient. Optimized maps for different hearing scenarios can be created, and front-end signal enhancement features can be also be included in the optimization procedure. In specific embodiments, the calculation of an optimized map can take place with a remote server where different sound and patients' current map can be stored, or maybe a simplified model is utilised in a mobile device, e.g. the remote control, or in the sound processor unit itself. The sounds used to produce the optimized map can be personalized by asking the patients to submit the sound environment where the patient usually stays. The calculation of the optimized map can also use an average profile for a specific listening environment.

Embodiments of the invention may be implemented in part in any conventional computer programming language. For example, preferred embodiments may be implemented in a procedural programming language (*e.g*., "C") or an object oriented programming language (*e.g*., "C++", Python). Alternative embodiments of the invention may be implemented as pre-programmed hardware elements, other related components, or as a combination of hardware and software components.

Embodiments can be implemented in part as a computer program product for use with a computer system. Such implementation may include a series of computer instructions fixed either on a tangible medium, such as a computer readable medium (*e.g*., a diskette, CD-ROM, ROM, or fixed disk) or transmittable to a computer system, via a modem or other interface device, such as a communications adapter connected to a network over a medium. The medium may be either a tangible medium (*e*.*g*., optical or analog communications lines) or a medium implemented with wireless techniques (*e*.*g*., microwave, infrared or other transmission techniques). The series of computer instructions embodies all or part of the functionality previously described herein with respect to the system. Those skilled in the art should appreciate that such computer instructions can be written in a number of programming languages for use with many computer architectures or operating systems. Furthermore, such instructions may be stored in any memory device, such as semiconductor, magnetic, optical or other memory devices, and may be transmitted using any communications technology, such as optical, infrared, microwave, or other transmission technologies. It is expected that such a computer program product may be distributed as a removable medium with accompanying printed or electronic documentation (*e.g*., shrink wrapped software), preloaded with a computer system (*e.g*., on system ROM or fixed disk), or distributed from a server or electronic bulletin board over the network (*e.g*., the Internet or World Wide Web). Of course, some embodiments of the invention may be implemented as a combination of both software (*e.g*., a computer program product) and hardware. Still other embodiments of the invention are implemented as entirely hardware, or entirely software (*e.g*., a computer program product).

Although various exemplary embodiments of the invention have been disclosed, it should be apparent to those skilled in the art that various changes and modifications can be made which will achieve some of the advantages of the invention without departing from the true scope of the invention.

## Claims

1. A method of fitting an implanted patient with a hearing implant system having an implanted electrode array with a plurality of electrode contacts, the method comprising:
determining, based on objective response measurement results, a preliminary fit map that characterizes preliminary patient-specific operating parameters for the hearing implant system;
and
producing at least one adjusted fit map that characterizes adjusted patient-specific operating parameters for the hearing implant system, wherein said producing said at least one adjusted fit map comprises the following steps:
- varying said preliminary patient-specific operating parameters such as to provide a plurality of different fit maps with the constraint that the overall loudness remains similar,
- for each of said plurality of different fit maps, utilizing an electric stimulation neural response model (503) to provide a corresponding electric stimulation neural response pattern (505) using cochlear implant electrical stimulation patterns (513) from a speech/sound database (501), wherein map parameters in the cochlear implant electric simulation patterns (513) are varied according to said different fit maps,
- utilizing a normal hearing neural response model (502) to provide an acoustic stimulation neural response pattern (504) using data from said speech/sound database (501),
- comparing said electric stimulation neural response patterns (505) corresponding to said plurality of different fit maps with said acoustic stimulation neural response pattern (504), and choosing, as the at least one adjusted fit map, the fit map for which the electric stimulation neural response pattern (505) is closest to the acoustic stimulation neural response pattern (504).

2. The method according to claim 1, wherein the at least one adjusted fit map comprises a plurality of adjusted fit maps, each corresponding to a different hearing environment.

3. The method according to claim 1, wherein the preliminary fit map further reflects subjective feedback from the implanted patient.

4. The method according to claim 1, wherein said preliminary patient-specific operating parameters are varied with the further constraint that speech or phoneme recognition rate remain similar.

5. The method according to claim 1, said method comprising using a parameter adjustment algorithm to change the patient-specific operating parameters.

6. The method according to claim 5, wherein the parameter adjustment algorithm applies a geometric shaping to the preliminary fit map.

7. A non-transitory tangible computer-readable medium having instructions thereon for fitting an implanted patient and a hearing implant system having an implanted electrode array with a plurality of electrode contacts, the instructions comprising:
performing objective response measurements following delivery of preliminary electrical stimulation signals to the electrode contacts;
based on the measurements, determining a preliminary fit map that characterizes preliminary patient-specific operating parameters for the hearing implant system; and
producing at least one adjusted fit map that characterizes adjusted patient-specific operating parameters for the hearing implant system, wherein said producing said at least one adjusted fit map comprises the following steps:
- varying said preliminary patient-specific operating parameters such as to provide a plurality of different fit maps with the constraint that the overall loudness remains similar,
- for each of said plurality of different fit maps, utilizing an electric stimulation neural response model (503) to provide an electric stimulation neural response pattern (505) using cochlear implant electrical stimulation patterns (513)from a speech/sound database (501), wherein map parameters in the cochlear implant electric simulation patterns (513) are varied according to said different fit maps,
- utilizing a normal hearing neural response model (502) to provide an acoustic stimulation neural response pattern (504) using data from said speech/sound database (501),
- comparing said electric stimulation neural response patterns (505) corresponding to said plurality of different fit maps with said acoustic stimulation neural response pattern (504), and choosing, as the at least one adjusted fit map, the fit map for which the electric stimulation neural response pattern (505) is closest to the acoustic stimulation neural response pattern (504).

8. The computer-readable medium according to claim 7, wherein the at least one adjusted fit map comprises a plurality of adjusted fit maps, each corresponding to a different hearing environment.

9. The computer-readable medium according to claim 7, wherein the preliminary fit map further reflects subjective feedback from the implanted patient.

10. The computer-readable medium according to claim 7, wherein said preliminary patient-specific operating parameters are varied with the further constraint that speech or phoneme recognition rate remain similar.

11. The computer-readable medium according to claim 7, wherein using the preliminary fit map to constrain an implant neural response model includes using a parameter adjustment algorithm to change the patient-specific operating parameters.

12. The computer-readable medium according to claim 11, wherein the parameter adjustment algorithm applies a geometric shaping to the preliminary fit.

## Patentansprüche

1. Verfahren zum Anpassen eines implantierten Patienten mit einem Hörimplantatsystem, das eine implantierte Elektrodenanordnung mit einer Vielzahl von Elektrodenkontakten aufweist, wobei das Verfahren Folgendes umfasst:
Bestimmen, basierend auf objektiven Antwortmessergebnissen, einer vorläufigen Anpassungszuordnung, die vorläufige patientenspezifische Betriebsparameter für das Hörimplantatsystem charakterisiert;
und
Erzeugen mindestens einer angepassten Anpassungszuordnung, die angepasste patientenspezifische Betriebsparameter für das Hörimplantatsystem charakterisiert, wobei das Erzeugen der mindestens einen angepassten Anpassungszuordnung die folgenden Schritte umfasst:
- Variieren der vorläufigen patientenspezifischen Betriebsparameter, um eine Vielzahl von unterschiedlichen Anpassungszuordnungen unter der Maßgabe bereitzustellen, dass die Gesamtlautstärke ähnlich bleibt,
- für jede der Vielzahl von unterschiedlichen Anpassungszuordnungen, Verwenden eines Elektrische-Stimulation-Neuralantwortmodells (503), um ein entsprechendes Elektrische-Stimulation-Neuralantwortmuster (505) unter Verwendung elektrischer Cochlea-Implantat-Stimulationsmuster (513) aus einer Sprach-/Tondatenbank (501) bereitzustellen, wobei Zuordnungsparameter in den elektrischen Cochlea-Implantat-Simulationsmustern (513) gemäß den unterschiedlichen Anpassungszuordnungen variiert werden,
- Verwenden eines Normalhör-Neuralantwortmodells (502), um ein Akustische-Stimulation-Neuralantwortmuster (504) unter Verwendung von Daten aus der Sprach-/Tondatenbank (501) bereitzustellen,
- Vergleichen der Elektrische-Stimulation-Neuralantwortmuster (505), die der Vielzahl von unterschiedlichen Anpassungszuordnungen entsprechen, mit dem Akustische-Stimulation-Neuralantwortmuster (504), und Auswählen der Anpassungszuordnung, für die das Elektrische-Stimulation-Neuralantwortmuster (505) dem Akustische-Stimulation-Neuralantwortmuster (504) am nächsten ist, als die mindestens eine angepasste Anpassungszuordnung.

2. Verfahren nach Anspruch 1, wobei die mindestens eine angepasste Anpassungszuordnung eine Vielzahl von angepassten Anpassungszuordnungen umfasst, die jeweils einer unterschiedlichen Hörumgebung entsprechen.

3. Verfahren nach Anspruch 1, wobei die vorläufige Anpassungszuordnung ferner eine subjektive Rückmeldung von dem implantierten Patienten widerspiegelt.

4. Verfahren nach Anspruch 1, wobei die vorläufigen patientenspezifischen Betriebsparameter unter der weiteren Maßgabe mvariiert werden, dass die Sprach- oder Phonemerkennungsrate ähnlich bleibt.

5. Verfahren nach Anspruch 1, wobei das Verfahren das Verwenden eines Parameteranpassungsalgorithmus umfasst, um die patientenspezifischen Betriebsparameter zu ändern.

6. Verfahren nach Anspruch 5, wobei der Parameteranpassungsalgorithmus eine geometrische Formung auf die vorläufige Anpassungszuordnung anwendet.

7. Nichtflüchtiges greifbares computerlesbares Medium mit Anweisungen darauf zum Anpassen eines implantierten Patienten und eines Hörimplantatsystems, das eine implantierte Elektrodenanordnung mit einer Vielzahl von Elektrodenkontakten aufweist, wobei die Anweisungen Folgendes umfassen:
Durchführen von objektiven Antwortmessungen nach der Abgabe von vorläufigen elektrischen Stimulationssignalen an die Elektrodenkontakte;
basierend auf den Messungen, Bestimmen einer vorläufigen Anpassungszuordnung, die vorläufige patientenspezifische Betriebsparameter für das Hörimplantatsystem charakterisiert; und
Erzeugen mindestens einer angepassten Anpassungszuordnung, die angepasste patientenspezifische Betriebsparameter für das Hörimplantatsystem charakterisiert, wobei das Erzeugen der mindestens einen angepassten Anpassungszuordnung die folgenden Schritte umfasst:
- Variieren der vorläufigen patientenspezifischen Betriebsparameter, um eine Vielzahl von unterschiedlichen Anpassungszuordnungen unter der Maßgabe bereitzustellen, dass die Gesamtlautstärke ähnlich bleibt,
- für jede der Vielzahl von unterschiedlichen Anpassungszuordnungen, Verwenden eines Elektrische-Stimulation-Neuralantwortmodells (503), um ein Elektrische-Stimulation-Neuralantwortmuster (505) unter Verwendung elektrischer Cochlea-Implantat-Stimulationsmuster (513) aus einer Sprach-/Tondatenbank (501) bereitzustellen, wobei Zuordnungsparameter in den elektrischen Cochlea-Implantat-Simulationsmustern (513) gemäß den unterschiedlichen Anpassungszuordnungen variiert werden,
- Verwenden eines Normalhör-Neuralantwortmodells (502), um ein Akustische-Stimulation-Neuralantwortmuster (504) unter Verwendung von Daten aus der Sprach-/Tondatenbank (501) bereitzustellen,
- Vergleichen der Elektrische-Stimulation-Neuralantwortmuster (505), die der Vielzahl von unterschiedlichen Anpassungszuordnungen entsprechen, mit dem Akustische-Stimulation-Neuralantwortmuster (504), und Auswählen der Anpassungszuordnung, für die das Elektrische-Stimulation-Neuralantwortmuster (505) dem Akustische-Stimulation-Neuralantwortmuster (504) am nächsten ist, als die mindestens eine angepasste Anpassungszuordnung.

8. Computerlesbares Medium nach Anspruch 7, wobei die mindestens eine angepasste Anpassungszuordnung eine Vielzahl von angepassten Anpassungszuordnungen umfasst, die jeweils einer unterschiedlichen Hörumgebung entsprechen.

9. Computerlesbares Medium nach Anspruch 7, wobei die vorläufige Anpassungszuordnung ferner eine subjektive Rückmeldung von dem implantierten Patienten widerspiegelt.

10. Computerlesbares Medium nach Anspruch 7, wobei die vorläufigen patientenspezifischen Betriebsparameter unter der weiteren Maßgabe variiert werden, dass die Sprach- oder Phonemerkennungsrate ähnlich bleibt.

11. Computerlesbares Medium nach Anspruch 7, wobei das Verwenden der vorläufigen Anpassungszuordnung, um ein Implantat-Neuralantwortmodell einzuschränken, das Verwenden eines Parameteranpassungsalgorithmus umfasst, um die patientenspezifischen Betriebsparameter zu ändern.

12. Computerlesbares Medium nach Anspruch 11, wobei der
Parameteranpassungsalgorithmus eine geometrische Formung auf die vorläufige Anpassung anwendet.

## Revendications

1. Procédé d'ajustement d'un patient implanté avec un système d'implant auditif comprenant un réseau d'électrodes implanté ayant une pluralité de contacts d'électrode, le procédé comprenant : déterminer, sur la base de résultats de mesure de réponse objective, une carte d'ajustement préliminaire qui caractérise des paramètres de fonctionnement spécifiques au patient préliminaires pour le système d'implant auditif ;
et
produire au moins une carte d'ajustement modifiée qui caractérise des paramètres de fonctionnement spécifiques au patient modifiés pour le système d'implant auditif, la production de ladite au moins une carte d'ajustement modifiée comprenant les étapes suivantes :
- faire varier lesdits paramètres de fonctionnement spécifiques au patient préliminaires de manière à fournir une pluralité de cartes d'ajustement différentes avec la contrainte que le niveau sonore global reste similaire,
- pour chacune de ladite pluralité de cartes d'ajustement différentes, utiliser un modèle de réponse neuronale à la stimulation électrique (503) pour fournir un schéma de réponse neuronale à la stimulation électrique (505) à l'aide de schémas de stimulation électrique d'implant cochléaire (513) issus d'une base de données de parole/sons (501), les paramètres de carte dans les schémas de stimulation électrique d'implant cochléaire (513) étant modifiés selon lesdites cartes d'ajustement différentes,
- utiliser un modèle de réponse neuronale d'audition normale (502) pour fournir un schéma de réponse neuronale à la stimulation acoustique (504) à l'aide de données provenant de ladite base de données de parole/sons (501),
- comparer lesdits schémas de réponse neuronale à la stimulation électrique (505) correspondant à ladite pluralité de cartes d'ajustement différentes avec ledit schéma de réponse neuronale à la stimulation acoustique (504), et choisir, comme la au moins une carte d'ajustement modifiée, la carte d'ajustement pour laquelle le schéma de réponse neuronale à la stimulation électrique (505) est le plus proche du schéma de réponse neuronale à la stimulation acoustique (504).

2. Procédé selon la revendication 1, dans lequel ladite au moins une carte d'ajustement modifiée comprend une pluralité de cartes d'ajustement modifiées, chacune correspondant à un environnement auditif différent.

3. Procédé selon la revendication 1, dans lequel la carte d'ajustement préliminaire reflète en outre le retour subjectif du patient implanté.

4. Procédé selon la revendication 1, dans lequel lesdits paramètres de fonctionnement spécifiques au patient préliminaires sont modifiés avec la contrainte supplémentaire que le taux de reconnaissance de la parole ou des phonèmes reste similaire.

5. Procédé selon la revendication 1, ledit procédé comprenant l'utilisation d'un algorithme d'ajustement de paramètre pour modifier les paramètres de fonctionnement spécifiques au patient.

6. Procédé selon la revendication 5, dans lequel l'algorithme d'ajustement de paramètre applique une mise en forme géométrique à la carte d'ajustement préliminaire.

7. Support informatique non transitoire lisible par ordinateur comportant des instructions pour l'ajustement d'un patient implanté et d'un système d'implant auditif comprenant un réseau d'électrodes implanté ayant une pluralité de contacts d'électrode, les instructions comprenant :
effectuer des mesures de réponse objective après la délivrance de signaux de stimulation électrique préliminaires aux contacts d'électrode ;
sur la base des mesures, déterminer une carte d'ajustement préliminaire qui caractérise des paramètres de fonctionnement spécifiques au patient préliminaires pour le système d'implant auditif ; et produire au moins une carte d'ajustement modifiée qui caractérise des paramètres de fonctionnement spécifiques au patient modifiés pour le système d'implant auditif, la production de ladite au moins une carte d'ajustement modifiée comprenant les étapes suivantes :
- faire varier lesdits paramètres de fonctionnement spécifiques au patient préliminaires de manière à fournir une pluralité de cartes d'ajustement différentes avec la contrainte que le niveau sonore global reste similaire,
- pour chacune de ladite pluralité de cartes d'ajustement différentes, utiliser un modèle de réponse neuronale à la stimulation électrique (503) pour fournir un schéma de réponse neuronale à la stimulation électrique (505) à l'aide de schémas de stimulation électrique d'implant cochléaire (513) issus d'une base de données de parole/sons (501), les paramètres de carte dans les schémas de stimulation électrique d'implant cochléaire (513) étant modifiés selon lesdites cartes d'ajustement différentes,
- utiliser un modèle de réponse neuronale d'audition normale (502) pour fournir un schéma de réponse neuronale à la stimulation acoustique (504) à l'aide de données provenant de ladite base de données de parole/sons (501),
- comparer lesdits schémas de réponse neuronale à la stimulation électrique (505) correspondant à ladite pluralité de cartes d'ajustement différentes avec ledit schéma de réponse neuronale à la stimulation acoustique (504), et choisir, comme la au moins une carte d'ajustement modifiée, la carte d'ajustement pour laquelle le schéma de réponse neuronale à la stimulation électrique (505) est le plus proche du schéma de réponse neuronale à la stimulation acoustique (504).

8. Support informatique lisible par ordinateur selon la revendication 7, dans lequel ladite au moins une carte d'ajustement modifiée comprend une pluralité de cartes d'ajustement modifiées, chacune correspondant à un environnement auditif différent.

9. Support informatique lisible par ordinateur selon la revendication 7, dans lequel la carte d'ajustement préliminaire reflète en outre le retour subjectif du patient implanté.

10. Support informatique lisible par ordinateur selon la revendication 7, dans lequel lesdits paramètres de fonctionnement spécifiques au patient préliminaires sont modifiés avec la contrainte supplémentaire que le taux de reconnaissance de la parole ou des phonèmes reste similaire.

11. Support informatique lisible par ordinateur selon la revendication 7, dans lequel l'utilisation de la carte d'ajustement préliminaire pour contraindre un modèle de réponse neuronale d'implant comprend l'utilisation d'un algorithme d'ajustement de paramètre pour modifier les paramètres de fonctionnement spécifiques au patient.

12. Support informatique lisible par ordinateur selon la revendication 11, dans lequel l'algorithme d'ajustement de paramètre applique une mise en forme géométrique à la carte d'ajustement préliminaire.
